Europäisches Patentamt
European Patent Office
Office européen des brevets

(19)

(11) Veröffentlichungsnummer: **0 173 323**
**A1**

(12) **EUROPÄISCHE PATENTANMELDUNG**

(21) Anmeldenummer: **85110837.3**

(22) Anmeldetag: **19.08.85**

(51) Int. Cl.⁴: **C 07 D 401/12**, C 07 D 401/14, C 07 D 403/12, C 07 D 403/14, C 07 D 405/14, C 07 D 409/14, C 07 D 413/12, C 07 D 413/14, C 07 D 417/12, C 07 D 417/14, C 07 D 419/14

(30) Priorität: **30.08.84 DE 3431926**

(43) Veröffentlichungstag der Anmeldung: **05.03.86 Patentblatt 86/10**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Diehr, Hans-Joachim, Dr., Höhe 35, D-5600 Wuppertal 1 (DE)**
Erfinder: **Fest, Christa, Dr., Im Johannistal 20, D-5600 Wuppertal 1 (DE)**
Erfinder: **Kirsten, Rolf, Dr., Carl-Langhans-Strasse 27, D-4019 Monheim (DE)**
Erfinder: **Kluth, Joachim, Dr., Kurt-Schumacher-Strasse 9, D-4018 Langenfeld (DE)**
Erfinder: **Müller, Klaus-Helmut, Dr., Bockhackstrasse 55, D-4000 Düsseldorf 13 (DE)**
Erfinder: **Pfister, Theodor, Dr., Lichtenberger Strasse 30, D-4019 Monheim (DE)**
Erfinder: **Priesnitz, Uwe, Dr., Severinstrasse 58, D-5650 Solingen 1 (DE)**
Erfinder: **Riebel, Hans-Jochem, Dr., In der Beek 92, D-5600 Wuppertal 1 (DE)**
Erfinder: **Roy, Wolfgang, Dr., Walter-Kolb-Strasse 47, D-4018 Langenfeld (DE)**
Erfinder: **Santel, Hans-Joachim, Dr., Gerstenkamp 19, D-5000 Köln 80 (DE)**
Erfinder: **Schmidt, Robert R. Dr., Im Waldwinkel 110, D-5060 Bergisch-Gladbach 2 (DE)**

(54) **Amidinoazole.**

(57) Die Erfindung betrifft neue Amidinoazole der allgemeinen Formel (I)

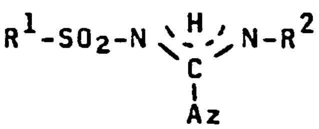

(I)

in welcher

$R^1$ für einen gegebenenfalls substituierten Rest aus der Reihe Alkyl, Aralkyl, Aryl und Heteroaryl steht,

$R^2$ für einen gegebenenfalls substituierten und/oder gegebenenfalls anellierten sechsgliedrigen aromatischen Heterocyclus, welcher wenigstens ein Stickstoffatom enthält, steht und

Az für einen gegebenenfalls substituierten Azolrest, d.h. einen fünfgliedrigen aromatischen Heterocyclus, welcher wenigstens ein Stickstoffatom enthält, steht, sowie Metallsalze von Verbindungen der Formel (I) und Addukte von Verbindungen der Formel (I) mit starken Säuren, ein erfinderisches Verfahren zu ihrer Herstellung und ihre Verwendung als Herbizide.

EP 0 173 323 A1

0 173 323

BAYER AKTIENGESELLSCHAFT 5090 Leverkusen, Bayerwerk
Konzernverwaltung RP Bi/mm
Patentabteilung IVb

Amidinoazole

Die Erfindung betrifft neue Amidinoazole, ein erfinderisches Verfahren zu ihrer Herstellung und ihre Verwendung als Herbizide.

Verschiedene Amidinoazol-Derivate sind als potentielle Herbizide bekannt geworden, haben jedoch bisher als Mittel zur Unkrautbekämpfung und/oder Regulierung des Pflanzenwachstums keine größere Bedeutung erlangt (vergl. DE-PS 2 321 330).

Es wurden nun neue Amidinoazole der allgemeinen Formel (I)

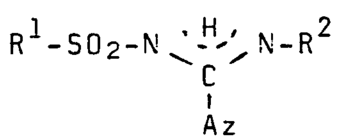

(I)

in welcher

$R^1$ für einen gegebenenfalls substituierten Rest aus der Reihe Alkyl, Aralkyl, Aryl und Heteroaryl steht,

$R^2$ für einen gegebenenfalls substituierten und/oder gegebenenfalls anellierten sechsgliedrigen aromatischen Heterocyclus, welcher wenigstens ein Stickstoffatom enthält, steht und

Az für einen gegebenenfalls substituierten Azolrest, d. h. einen fünfgliedrigen aromatischen Heterocyclus, welcher wenigstens ein Stickstoffatom enthält, steht,

sowie Metallsalze von Verbindungen der Formel (I) und Addukte von Verbindungen der Formel (I) mit starken Säuren gefunden.

Die allgemeine Formel (I) steht für die einzelnen Tautomeren der Formeln (IA) und (IB)

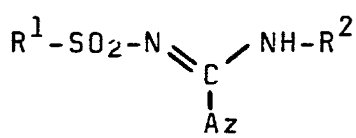

(IA)

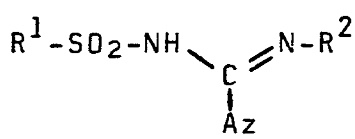

(IB),

in welcher

$R^1$, $R^2$ und Az die oben angegebenen Bedeutungen haben, sowie für Gemische der Tautomeren (IA) und (IB).

Das Mischungsverhältnis (IA)/(IB) hängt von aggregationsbestimmenden Faktoren, wie z. B. Temperatur, Lösungsmittel und Konzentration ab.

Man erhält die neuen Amidinoazole der Formel (I), nach einem erfinderischen Verfahren, wenn man Sulfonylguanidin-Derivate der Formel (II)

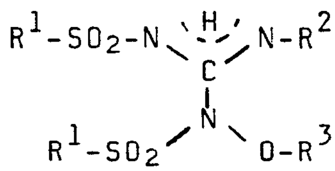

(II)

in welcher

$R^1$ und $R^2$ die oben angegebenen Bedeutungen haben und

$R^3$ für einen gegebenenfalls substituierten Kohlenwasserstoff-Rest steht,

mit Verbindungen der Formel (III)

M-Az (III)

in welcher

Az die oben angegebene Bedeutung hat und

M für Wasserstoff oder ein Metalläquivalent steht,

gegebenenfalls in Gegenwart von Basen und in Gegenwart von Verdünnungsmitteln bei Temperaturen zwischen 0 °C und 100 °C umsetzt und gegebenenfalls die hierbei erhaltenen

Produkte der Formel (I) mit Metallverbindungen bzw. mit Säuren behandelt.

Die neuen Amidinoazole der Formel (I), ihre Metallsalze und ihre Addukte mit starken Säuren zeichnen sich durch starke herbizide Wirksamkeit aus.

Ueberraschenderweise zeigen die neuen Verbindungen der Formel (I) erheblich bessere herbizide Wirkung als vorbekannte Amidinoazol-Derivate gleicher Wirkungsrichtung.

Es ist weiter als überraschend anzusehen, daß die erfindungsgemäßen Verbindungen der Formel (I) durch selektive Spaltung von Sulfonylguanidin-Derivaten der Formel (II) hergestellt werden können, da neben dieser neuartigen Reaktion auch andere Spaltungsreaktionen, beispielweise durch Angriff an den Sulfonyl-Gruppierungen, zu erwarten waren.

Gegenstand der Erfindung sind vorzugsweise Verbindungen der Formel (I), in welcher

$R^1$ für den Rest 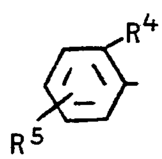 steht, worin

$R^4$ und $R^5$ gleich oder verschieden sind und für Wasserstoff, Halogen [wie insbesondere Fluor, Chlor und/oder Brom], Cyano, Nitro, $C_1$-$C_6$-Alkyl [welches gegebenenfalls durch Fluor, Chlor, Brom, Cyano, Carboxy, $C_1$-$C_4$-Alkoxycarbonyl, $C_1$-$C_4$-Alkylamino-carbonyl, Di-($C_1$-$C_4$-alkyl)-amino-carbonyl, Hydroxy, $C_1$-$C_4$-Alkoxy, Formyloxy, $C_1$-$C_4$-Alkyl-car-

bonyloxy, $C_1$-$C_4$-Alkoxy-carbonyloxy, $C_1$-$C_4$-Alkylamino-carbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)-aminosulfonyl, $C_3$-$C_6$-Cycloalkyl oder Phenyl substituiert ist], für $C_2$-$C_6$-Alkenyl [welches gegebenenfalls durch Fluor, Chlor, Brom, Cyano, $C_1$-$C_4$-Alkoxy-carbonyl, Carboxy oder Phenyl substituiert ist], für $C_1$-$C_4$-Alkoxy [welches gegebenenfalls durch Fluor, Chlor, Brom, Cyano, Carboxy, $C_1$-$C_4$-Alkoxy-carbonyl, $C_1$-$C_4$-Alkylthio,$C_1$-$C_4$-Alkylsulfinyl oder $C_1$-$C_4$-Alkylsulfonyl substituiert ist], für $C_3$-$C_6$-Alkenoxy [welches gegebenenfalls durch Fluor, Chlor, Brom, Cyano oder $C_1$-$C_4$-Alkoxy-carbonyl substituiert ist], $C_3$-$C_6$-Alkinoxy, für den Rest $-S(O)_p-R^6$, wobei

p für die Zahlen Null, 1 oder 2 steht und

$R^6$ für $C_1$-$C_4$-Alkyl [welches gegebenenfalls durch Fluor, Chlor, Brom, Cyano oder $C_1$-$C_4$-Alkoxy-carbonyl substituiert ist], $C_3$-$C_6$-Alkenyl, $C_3$-$C_6$-Alkinyl, $C_1$-$C_4$-Alkoxy, $C_1$-$C_4$-Alkoxyamino, $C_1$-$C_4$-Alkoxy-$C_1$-$C_4$-alkyl-amino, $C_1$-$C_4$-Alkylamino oder Di-($C_1$-$C_4$-alkyl)-amino steht,

$R^4$ und $R^5$ für Phenyl oder Phenoxy, für $C_1$-$C_4$-Alkyl-carbonylamino, $C_1$-$C_4$-Alkoxy-carbonylamino, $C_1$-$C_4$-Alkylamino-carbonyl-amino, Di-($C_1$-$C_4$-alkyl)-amino-carbonylamino, für den Rest -CO-$R^7$, wobei

$R^7$ für $C_1$-$C_6$-Alkyl, $C_1$-$C_6$-Alkoxy, $C_3$-$C_6$-Cycloalkoxy, $C_3$-$C_6$-Alkenoxy, $C_1$-$C_4$-Alkylthio, $C_1$-$C_4$-Alkylamino, $C_1$-$C_4$-Alkoxyamino, $C_1$-$C_4$-Alkoxy-$C_1$-$C_4$-alkyl-amino oder Di-($C_1$-$C_4$-alkyl)-amino steht (welche gegebenenfalls durch Fluor und/oder Chlor substituiert sind), oder

für $C_1$-$C_4$-Alkylsulfonyloxy, Di-($C_1$-$C_4$-al-kyl)-aminosulfonylamino oder für den Rest $-\underset{R^9}{\underset{|}{C}}{=}N-R^8$ stehen, wobei

$R^8$ für gegebenenfalls durch Fluor, Chlor, Cyano, Carboxy, $C_1$-$C_4$-Alkoxy, Carbonyl, $C_1$-$C_4$-Alkylthio, $C_1$-$C_4$-Alkylsulfinyl oder $C_1$-$C_4$-Alkylsulfonyl substituiertes $C_1$-$C_6$-Alkyl, für gegebenenfalls durch Fluor oder Chlor substituiertes Benzyl, für gegebenenfalls durch Fluor oder Chlor substituiertes $C_3$-$C_6$-Alkenyl oder $C_3$-$C_6$-Alkinyl, für gegebenenfalls durch Fluor, Chlor, Brom, $C_1$-$C_4$-Alkyl, $C_1$-$C_4$-Alkoxy, Trifluormethyl, Trifluormethoxy oder Trifluormethylthio substituiertes Phenyl, für gegebenenfalls durch Fluor und/oder Chlor substituiertes $C_1$-$C_6$-Alkoxy, $C_3$-$C_6$-Alkenoxy, $C_3$-$C_6$-Alkinoxy oder

Benzyloxy, für Amino, $C_1$-$C_4$-Alkylamino, Di-($C_1$-$C_4$-alkyl)-amino, Phenylamino, $C_1$-$C_4$-Alkyl-carbonyl-amino, $C_1$-$C_4$-Alkoxy-carbonylamino, $C_1$-$C_4$-Alkylsulfonylamino oder für gegebenenfalls durch Fluor, Chlor, Brom oder Methyl substituiertes Phenylsulfonylamino steht und

$R^9$ für Wasserstoff oder $C_1$-$C_4$-Alkyl steht;

worin weiter

$R^1$ für den Rest 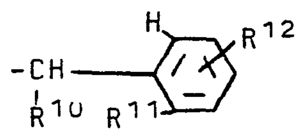 steht, worin

$R^{10}$ für Wasserstoff oder $C_1$-$C_3$-Alkyl steht,

$R^{11}$ und $R^{12}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], Carboxy, $C_1$-$C_4$-Alkoxy-carbonyl, $C_1$-$C_4$-Alkylsulfonyl oder Di-($C_1$-$C_4$-alkyl)-aminosulfonyl stehen; worin weiter

$R^1$ für den Rest 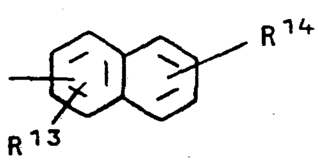 steht, worin

$R^{13}$ und $R^{14}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist] oder $C_1$-$C_4$-Alkoxy [welches gegebenenfalls durch Fluor und/oder Chlor substituiert ist], stehen; worin weiter

$R^1$ für den Rest 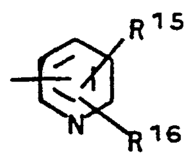 steht, worin

$R^{15}$ und $R^{16}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], für $C_1$-$C_4$-Alkylthio, $C_1$-$C_4$-Alkylsulfinyl oder $C_1$-$C_4$-Alkylsulfonyl [welche gegebenenfalls durch Fluor und/oder Chlor substituiert sind], sowie für Di-($C_1$-$C_4$-alkyl)-aminosulfonyl oder $C_1$-$C_4$-Alkoxy-carbonyl stehen; worin weiter

$R^1$ für den Rest 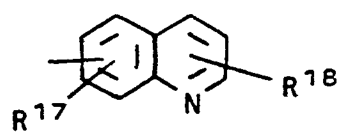 steht, worin

$R^{17}$ und $R^{18}$ gleich oder verschieden sind und für Wasserstoff, Fluor, Chlor, Brom, $C_1$-$C_4$-Alkyl [welches gegebenenfalls durch Fluor und/oder Brom substituiert ist], $C_1$-$C_4$-Alkoxy [welches gegebenenfalls durch Fluor und/oder Chlor substituiert ist], für $C_1$-$C_4$-Alkylthio, $C_1$-$C_4$-Alkylsulfinyl oder $C_1$-$C_4$-Alkylsulfonyl [welche gegebenenfalls durch Fluor und/oder Chlor substituiert sind] oder für Di-($C_1$-$C_4$-alkyl)-aminosulfonyl stehen; worin weiter

$R^1$ für den Rest 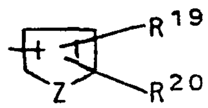 steht, worin

$R^{19}$ und $R^{20}$ gleich oder verschieden sind und für Wasserstoff, Fluor, Chlor, Brom, Cyano, Nitro, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylthio, $C_1$-$C_4$-Alkylsulfinyl oder $C_1$-$C_4$-Alkylsulfonyl [welches gegebenenfalls durch Fluor und/oder Chlor substituiert ist], Di-($C_1$-$C_4$-alkyl)-aminosulfonyl oder $C_1$-$C_4$-Alkoxy-carbonyl stehen, und

Z für Sauerstoff, Schwefel oder die Gruppierung N-$Z^1$ steht, wobei

$Z^1$ für Wasserstoff, $C_1$-$C_4$-Alkyl [welches

gegebenenfalls durch Fluor, Chlor, Brom oder Cyano substituiert ist], $C_3$-$C_6$-Cycloalkyl, Benzyl, Phenyl [welches gegebenenfalls durch Fluor, Chlor, Brom oder Nitro substituiert ist], $C_1$-$C_4$-Alkyl-carbonyl, $C_1$-$C_4$-Alkoxy-carbonyl oder Di-($C_1$-$C_4$-alkyl)-aminocarbonyl steht;

worin weiter

$R^2$ für den Rest 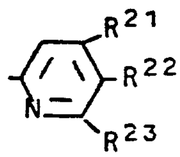 steht, worin

$R^{21}$ und $R^{23}$ gleich oder verschieden sind und für Wasserstoff, Fluor, Chlor, Brom, $C_1$-$C_4$-Alkyl [welches gegebenenfalls durch Fluor und/oder Chlor substituiert ist] oder $C_1$-$C_4$-Alkoxy [welches gegebenenfalls durch Fluor und/oder Chlor substiuiert ist] stehen mit der Maßgabe, daß wenigstens einer der Reste $R^{21}$ und $R^{23}$ von Wasserstoff verschieden ist, und

$R^{22}$ für Wasserstoff, Fluor, Chlor, Brom, Cyano oder $C_1$-$C_4$-Alkyl [welches gegebenenfalls durch Fluor und/oder Chlor substituiert ist] steht; worin weiter

$R^2$ für den Rest 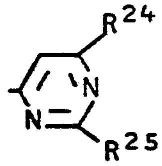 steht, worin

$R^{24}$ und $R^{25}$ gleich oder verschieden sind und für Wasserstoff, Fluor, Chlor, Brom, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylamino oder Di-($C_1$-$C_4$-alkyl)-amino stehen mit der Maßgabe, daß wenigstens einer der Reste $R^{24}$ und $R^{25}$ von Wasserstoff verschieden ist; worin weiter

$R^2$ für den Rest 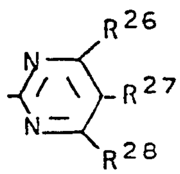 steht, worin

$R^{26}$ für Wasserstoff, Fluor, Chlor, Brom, Hydroxy, $C_1$-$C_4$-Alkyl [welches gegebenenfalls durch Fluor und/oder Chlor substituiert ist] oder $C_1$-$C_4$-Alkoxy [welches gegebenenfalls durch Fluor und/oder Chlor substituiert ist] steht,

$R^{27}$ für Wasserstoff, Fluor, Chlor, Brom, $C_1$-$C_4$-Alkyl [welches gegebenenfalls durch Fluor und/oder Chlor substituiert ist], Cyano, Formyl, $C_1$-$C_4$-Alkyl-carbonyl oder $C_1$-$C_4$-Alkoxy-carbonyl steht und

$R^{28}$ für Wasserstoff, Fluor, Chlor, Brom, Hydroxy, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], Amino, $C_1$-$C_4$-Alkyl-amino oder Di-($C_1$-$C_4$-alkyl)-amino steht, oder

$R^{27}$ und $R^{28}$ gemeinsam für $C_3$-$C_4$-Alkandiyl stehen; worin weiter

$R^2$ für den Rest 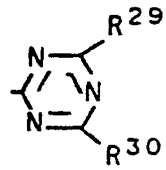 steht, worin

$R^{29}$ und $R^{30}$ gleich oder verschieden sind und für Fluor, Chlor, Brom, Hydroxy, $C_1$-$C_4$-Alkyl [welches gegebenenfalls durch Fluor und/oder Chlor substituiert ist], $C_3$-$C_5$-Cycloalkyl, $C_1$-$C_4$-Alkoxy [welches gegebenenfalls durch Fluor und/oder Chlor substituiert ist], $C_1$-$C_4$-Alkylthio oder für $C_1$-$C_4$-Alkyl-amino bzw. Di-($C_1$-$C_4$-Alkyl)-amino stehen; worin weiter

$R^2$ für den Rest 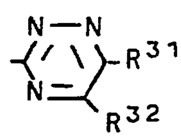 steht, worin

$R^{31}$ und $R^{32}$ gleich oder verschieden sind und für Wasserstoff, Methyl oder Methoxy stehen; worin weiter

Az für einen Rest aus der Reihe Pyrazol, Imidazol und Triazol steht, welcher gegebenenfalls durch Chlor, Brom, Jod, Nitro, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylthio [welches gegebenenfalls

durch Fluor und/oder Chlor substituiert ist] und/oder Phenyl [welches gegebenenfalls durch Fluor, Chlor, Brom, Jod, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio, Cyano, Nitro oder $C_1$-$C_4$-Alkoxycarbonyl substituiert ist] substituiert ist.

Gegenstand der Erfindung sind außerdem vorzugsweise Natrium-, Kalium- und Calciumsalze von Verbindungen der Formel (I) - wie sie vorausgehend vorzugsweise definiert sind. Diese Metallsalze werden erhalten durch Umsetzung von Verbindungen der Formel (I) mit geeigneten Metallverbindungen, insbesondere Metallalkoholaten, wie z.B. den Methylaten oder Ethylaten.

Gegenstand der Erfindung sind weiter vorzugsweise Addukte von Verbindungen der Formel (I) - wie vorausgehend definiert - mit Halogenwasserstoffsäuren, wie Hydrogenfluorid, Hydrogenchlorid, Hydrogenbromid, Hydrogeniodid, mit Schwefelsäure, mit gegebenenfalls durch Fluor und/oder Chlor substituierten Alkansulfonsäuren mit 1 bis 4 Kohlenstoffatomen oder auch Benzol- oder Naphthalinsulfonsäuren, welche gegebenenfalls durch Fluor, Chlor, Brom oder Methyl substituiert sind.

Gegenstand der Erfindung sind insbesondere Verbindungen der Formel (I), in welcher

(A) $R^1$ für den Rest 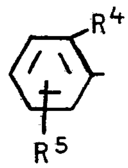 steht, worin

$R^4$ für Fluor, Chlor, Brom, Methyl, Chlormethyl, Trifluormethyl, Methoxy, Difluormethoxy, Trifluormethoxy, Methylthio, Difluormethylthio, Trifluormethylthio, Isopropylthio, Methylsulfinyl, Methylsulfonyl, Dimethylaminosulfonyl, $C_1$-$C_2$-Alkylaminosulfonyl, Phenyl, $C_1$-$C_3$-Alkoxy-carbonyl oder $C_3$-$C_6$-Cycloalkyloxycarbonyl steht und

$R^5$ für Wasserstoff steht; worin weiter

$R^2$ für den Rest 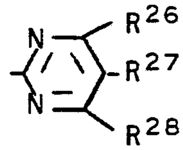 steht, worin

$R^{26}$ für Wasserstoff, Methyl, Hydroxy, Fluor, Chlor, Brom oder Difluormethoxy steht,

$R^{27}$ für Wasserstoff, Chlor, Brom oder Methyl steht und

$R^{28}$ für $C_1$-$C_3$-Alkyl, Hydroxy, Fluor, Chlor, Brom oder $C_1$-$C_3$-Alkoxy steht; worin weiter

Az für einen gegebenenfalls durch Methyl substituierten Pyrazol- oder Imidazol-Rest steht, oder in welcher

(B) Az sowie $R^1$ die oben unter (A) angegebenen Bedeutungen haben und

$R^2$ für den Rest 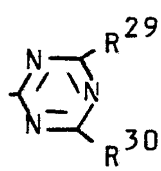 steht, worin

$R^{29}$ für Fluor, Chlor, Brom, Hydroxy, Methyl, Cyclopropyl, Methoxy, Ethoxy, Methylthio oder Ethylthio steht und

$R^{30}$ für Fluor, Chlor, Brom, Hydroxy, Methyl, Cyclopropyl, Methoxy, Ethoxy, Methylamino, Ethylamino, Dimethylamino oder Diethylamino steht.

Verwendet man beim erfindungsgemäßen Verfahren beispielsweise N'-(4,6-Dimethyl-pyrimidin-2-yl)-N''-methoxy-N''-N'''-bis-(2-methoxycarbonyl-benzolsulfonyl)-guanidin und Imidazol als Ausgangsstoffe, so kann der Reaktionsablauf durch folgendes Formelschema skizziert werden:

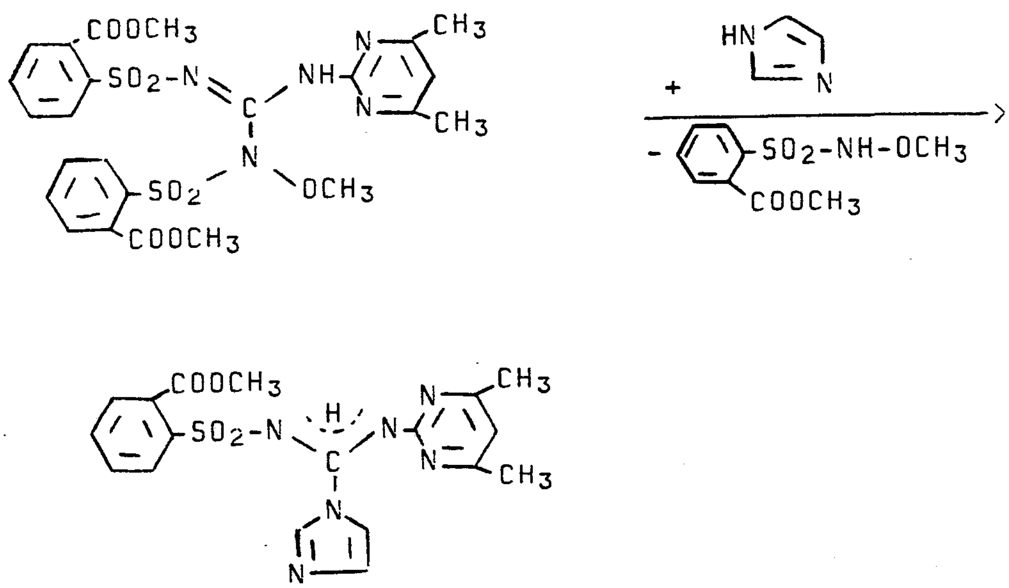

Die beim erfindungsgemäßen Herstellungsverfahren als Ausgangsstoffe zu verwendenden Sulfonylguanidin-Derivate sind durch die Formel (II) allgemein definiert. In dieser Formel haben vorzugsweise die Reste

$R^1$ und $R^2$ die gleichen Bedeutungen, wie sie oben im Rahmen der Definition der entsprechenden Reste in Formel (I) vorzugsweise genannt sind und

$R^3$ steht vorzugsweise für $C_1$-$C_4$-Alkyl oder Benzyl.

Insbesondere bevorzugt sind Ausgangsstoffe der Formel (II) in welcher die Reste

$R^1$ und $R^2$ die gleichen Bedeutungen haben, wie sie oben im Rahmen der Definition der entsprechenden Reste in Formel (I) als insbesondere bevorzugt genannt sind und

$R^3$ für Methyl steht.

Als Beispiele für die Ausgangsstoffe der Formel (II) seien genannt:

N'-(4,6-Dimethyl-pyrimidin-2-yl)-N''-methoxy-
-N'',N'''-bis-(2-chlor-benzolsulfonyl)-,
-N'',N'''-bis-(2-brom-benzolsulfonyl)-,
-N'',N'''-bis-(2-fluor-benzolsulfonyl)-,
-N'',N'''-bis-(2-methyl-benzolsulfonyl)-,
-N'',N'''-bis-(2-trifluormethyl-benzolsulfonyl)-,
-N'',N'''-bis-(2-methoxy-benzolsulfonyl)-,
-N'',N'''-bis-(2-phenyl-benzolsulfonyl)- und
-N'',N'''-bis-(2-methoxycarbonyl-benzolsulfonyl)-
-guanidin.

Die als Ausgangsstoffe zu verwendenden Sulfonyl-guanidin-Derivate der Formel (II) sind Gegenstand einer vorgängigen, jedoch nicht zum vorveröffentlichten Stand der Technik gehörenden Patentanmeldung der Anmelderin (vergl. DE-OS 3 334 455).

Man erhält die Verbindungen der Formel (II), wenn man Guanidin-Derivate der Formel (IV),

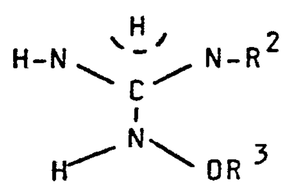

(IV)

in welcher

$R^2$ und $R^3$ die oben angegebenen Bedeutungen haben,

mit Sulfonsäurechloriden der Formel (V)

$R^1-SO_2-Cl$ (V)

in welcher

$R^1$ die oben angegebene Bedeutung hat,

in Gegenwart von Säureakzeptoren, wie z. B. Pyridin oder Diazabicyclooctan (DABCO), und gegebenenfalls in Gegenwart von Verdünnungsmitteln, wie z. B. Methylenchlorid oder Chloroform, bei Temperaturen zwischen -20 °C und +50 °C, vorzugsweise bei 0 bis 30 °C umsetzt. Die Aufarbeitung kann auf übliche Weise durchgeführt werden, beispielsweise durch Versetzen mit Wasser, gegebenenfalls Ansäuern z. B. mit Salzsäure, Extrahieren mit einem mit Wasser praktisch nicht mischbaren Lösungsmittel, wie z. B. Methylenchlorid oder Chloroform, Waschen der organischen Phase mit Wasser, Trocknen, Filtrieren und Einengen. Die dabei im Rückstand verbleibenden Produkte der Formel (II) können im allgemeinen mit organischen Lösungsmitteln zur Kristallisation gebracht und gegebenenfalls durch Umkristallisieren gereinigt werden.

Die als Zwischenprodukte benötigten Guanidin-Derivate der Formel (IV) sind ebenfalls Gegenstand der oben genannten vorgängigen Patentanmeldung der Anmelderin (vergl. DE-OS 3 334 455).

Man erhält diese Guanidin-Derivate, wenn man Cyanaminover-

bindungen der Formel (VI)

$$NC-NH-R^2 \qquad (VI)$$

in welcher

$R^2$ die oben angegebene Bedeutung hat,

mit Hydroxylamin-Derivaten der Formel (VII)

$$H_2N-OR^3 \qquad (VII)$$

in welcher

$R^3$ die oben angegebene Bedeutung hat,

bzw. deren Hydrochloriden

gegebenenfalls in Gegenwart von Verdünnungsmitteln, wie z. B. Ethanol oder Butanol, bei Temperaturen zwischen 20 °C und 150 °C, vorzugsweise zwischen 50 °C und 120 °C, umsetzt und gegebenenfalls anschließend mit Säureakzeptoren, wie z. B. (wässr.) Ammoniak, Natriumhydroxid oder Kaliumcarbonat, behandelt. Die Guanidin-Derivate (IV) fallen hierbei im allgemeinen kristallin an.

Die Cyanaminoverbindungen der Formel (VI) sind zum Teil bekannt (vergl. J. Chem. Soc. 1953, 1725 - 1730). Man erhält diese Verbindungen im wesentlichen nach folgenden zwei Synthesewegen:

(1) allgemein durch Umsetzung von Alkalimetall- oder Erdalkalimetall-Salzen von Cyanamid - wie z. B. Natriumcyanamid oder Calciumcyanamid - mit Halogenverbindungen

der Formel (VIII)

$$Hal^1-R^2 \qquad (VIII)$$

in welcher

$R^2$ die oben angegebene Bedeutung hat und

$Hal^1$ für Fluor, Chlor, Brom oder Jod, insbesondere für Chlor, steht,

gegebenenfalls in Gegenwart von inerten Verdünnungsmitteln, wie z. B. Aceton, Acetonitril oder Dimethylformamid, bei Temperaturen zwischen 0 °C und 150 °C, vorzugsweise zwischen 10 °C und 100 °C; nach Abdestillieren der flüchtigen Komponente und Auflösen des Rückstandes in Wasser können die Cyanaminoverbindungen der Formel (VI) durch Ansäuern, z. B. mit Salzsäure, ausgefällt und durch Absaugen isoliert werden; oder

(2) für den Fall, daß $R^2$ für einen substituierten Pyrimidinylrest steht, durch Umsetzung von Cyanoguanidin ('Dicyandiamid') mit ß-Dicarbonylverbindungen, wie z.B. Acetylaceton oder deren Derivaten, wie z.B. Acetalen oder Enaminen (vgl. J.Chem.Soc. 1953, 1725-1730), Acetessigsäureester (vgl. J. Prakt. Chem. 77, (1908), 542 und J. Chem. Soc. 1948, 586) oder Malonsäureestern (vergl. DE-PS 158 591). Die aus Acetessigsäureestern bzw. Malonsäureestern erhaltenen 2-Cyanamino-4-hydroxy-6-methyl- bzw. -4,6-di-hydroxy-pyrimidine können auf bekannte Weise durch Umsetzung mit Alkylierungsmitteln, wie z. B. Dimethyl- oder Diethylsulfat, gegebenenfalls in Gegenwart von Verdünnungsmitteln, wie z. B. Wasser, Methanol, Ethanol, n- und iso-Propanol, Aceton, Dioxan oder Dimethylformamid, und in Gegenwart von Säurebindemitteln, wie z. B.

Natrium- oder Kalium-hydroxid, Natrium- oder Kaliumcarbonat, in entsprechende 2-Cyanamino-4-alkoxy-6-methyl- bzw. -4,6-dialkoxy-pyrimidine umgewandelt werden. Zur Vermeidung einer N-Alkylierung wird gegebenenfalls mit einem Acylierungsmittel, wie z. B. Acetanhydrid oder Acetylchlorid acyliert und nach der Alkylierung wird mit wässrigen Säuren oder Basen wieder entacyliert.

Die Halogenverbindungen der Formel (VIII) sind bekannt (vergl. J. Chem. Soc. (C) 1966, 2031; Chem. Pharm. Bull. 11 (1963), 1382 - 1388; Arch. Pharm. 295 (1962), 649 - 657).

Die Hydroxylamin-Derivate der Formel (VII) sind bekannt oder können nach an sich bekannten Verfahren hergestellt werden (vergl. Chem. Pharm. Bull. 15 (1967), 345 - 349; Bull. Soc. Chim. France 1958 664; Synthesis 1976, 682).

Die Sulfonsäurechloride der Formel (V) sind zum Teil bekannt (vergl. Chemistry Lett. 1978, 951; EP-PA 23 422, 35 893, 42 731, 44 808, 44 809, 51 466, 64 804 und 70 041; US-PS 2 929 820, 4 282 242 und 4 372 778; J. Org. Chem. 33 (1968), 2104).
Man erhält diese Verbindungen im wesentlichen nach folgenden zwei Synthesewegen:

(1) durch Umsetzung der entsprechenden Sulfonsäuren $R^1SO_3H$ bzw. von deren Alkalimetall- oder Erdalkalimetall-Salzen mit Halogenierungsmitteln, wie z. B. Phosphor-(V)chlorid (Phosphorpentachlorid), Phosphorylchlorid (Phosphoroxychlorid), Thionylchlorid, Phosgen oder Benzotrichlorid, gegebenenfalls in Gegenwart von Katalysatoren, wie z. B. Pyridin oder Dimethylformamid,

und gegebenenfalls unter Verwendung von inerten Verdünnungsmitteln, wie z. B. Methylenchlorid, Chloroform, Acetonitril, Chlorbenzol und/oder Sulfolan bei Temperaturen zwischen -20 °C und +150 °C, vorzugsweise zwischen 0 °C und +100 °C; nach Verdünnen mit Wasser können die Sulfonsäurechloride - soweit sie kristallin anfallen - durch Absaugen isoliert werden oder durch Extrahieren mit einem mit Wasser nicht mischbaren Lösungsmittel, wie z. B. Methylenchlorid, Diethylether oder Hexan, Waschen und Trocknen der Extrakte, Einengen und Umkristallisieren bzw. Destillieren gereinigt werden; oder auch

(2) auf an sich bekannte Weise (vergl. J. Org. Chem. 25 (1960), 1824; DE-OS 2 308 262 und EP-PA 59 241) durch Umsetzung entsprechender Aminoverbindungen $R^1-NH_2$ mit Natriumnitrit und Salzsäure, gegebenenfalls in Gegenwart von Essigsäure, bei Temperaturen zwischen -10 °C und +20 °C, vorzugsweise zwischen -5 °C und +10 °C, und anschließend (in situ) mit Schwefeldioxid oder einem Salz der schwefeligen Säure, wie z. B. Natriumsulfit oder Natriumhydrogensulfit in Gegenwart einer Kupferverbindung, wie z. B. Kupferchlorid oder Kupfersulfat, als Katalysator bei Temperaturen zwischen 0 °C und 80 °C, vorzugsweise zwischen 10 °C und 60 °C.

Die Aufarbeitung kann auf übliche Weise erfolgen: Bei Verdünnen mit Wasser fallen die Sulfonsäurechloride im allgemeinen kristallin an und können durch Absaugen isoliert werden. Sie können aber auch aus der wässrigen Dispersion mit einem mit Wasser praktisch nicht mischbaren Lösungsmittel, wie z. B. Methylenchlorid oder Diethylether, extrahiert, getrocknet und durch Vakuumdestillation gereinigt werden.

Die beim erfindungsgemäßen Verfahren weiter als Ausgangsstoffe zu verwendenden Verbindungen sind durch die Formel (III) allgemein definiert. In dieser Formel hat vorzugsweise

Az die gleiche Bedeutung, wie sie oben im Rahmen der Substituentendefinition der Formel (I) vorzugsweise genannt ist und

M steht vorzugsweise für Wasserstoff, Lithium, Natrium oder Kalium.

Insbesondere bevorzugt sind Ausgangsstoffe der Formel (III), in welcher Az die gleiche Bedeutung hat, wie sie oben im Rahmen der Substituentendefinition der Formel (I) als insbesondere bevorzugt genannt ist und M für Wasserstoff steht.

Als Beispiele für die Ausgangsstoffe der Formel (III) seien genannt:

Pyrazol, 3,5-Dimethyl-pyrazol, 4-Chlor-pyrazol, 4-Brompyrazol, 4-Jod-pyrazol, 4-Chlor-3,5-dimethyl-pyrazol, Imidazol, 2-Methyl-imidazol, 2-Ethyl-imidazol, 2-Propyl-imidazol, 2-Isopropyl-imidazol, 4-Methyl-imidazol, 4-Ethylimidazol, 4-Nitro-imidazol, 2-Methyl-4-nitro-imidazol, 2-Methyl-5-nitro-imidazol, 4-Methyl-5-nitro-imidazol, 2,4-Dimethyl-imidazol und 1,2,4-Triazol.

Die als Ausgangsstoffe zu verwendenden Azole der Formel (III) sind bekannt.

Das erfindungsgemäße Verfahren wird vorzugsweise in Gegenwart von Verdünnungsmitteln durchgeführt. Als solche kommen praktisch alle inerten organischen Lösungsmittel, vorzugsweise jedoch aprotisch polare Solventien in Betracht. Hierzu gehören halogenierte Kohlenwasserstoffe, wie z. B. Methylenchlorid, Chloroform, 1,2-Dichlorethan und Chlorbenzol, Ketone, wie z. B. Aceton, Methylethylketon, Methylisopropylketon und Methylisobutylketon, Ester, wie z. B. Essigsäuremethylester und Essigsäureethylester, Nitrile, wie z. B. Acetonitril und Propionitril, Ether, wie z. B. Diethylether, Diisopropylether, 1,2-Dimethoxyethan, Tetrahydrofuran und Dioxan, Amide, wie z. B. Dimethylformamid und Dimethylacetamid, sowie Dimethylsulfoxid und Sulfolan.

Das erfindungsgemäße Verfahren wird vorzugsweise in Gegenwart von Basen durchgeführt. Es kommen die üblichen Säureakzeptoren in Betracht, vorzugsweise jedoch nucleophile Stickstoffverbindungen, welche praktisch keine oder nur sehr geringe Protonendonator-Eigenschaften aufweisen. Hierzu gehören Trialkylamine, wie z. B. Trimethylamin, Triethylamin, Tripropylamin und Tributylamin, Dialkyl-aralkylamine, wie z. B. N,N-Dimethyl-benzylamin und N,N-Diethylbenzylamin, Dialkyl-arylamine, wie z. B. N,N-Dimethylanilin und N,N-Diethyl-anilin, sowie Stickstoffheterocyclen, wie z. B. Pyridin, 2-Methyl-pyridin, 2-Methyl-5-ethyl-pyridin, 4-Dimethyl-amino-pyridin und Diazabicyclooctan (DABCO).

Die Reaktionstemperaturen können beim erfindungsgemäßen Verfahren innerhalb eines größeren Bereichs variiert werden. Im allgemeinen arbeitet man zwischen 0 °C und 100 °C,

vorzugsweise zwischen 10 °C und 80 °C.

Das erfindungsgemäße Verfahren wird im allgemeinen bei Normaldruck durchgeführt.

Zur Durchführung des erfindungsgemäßen Verfahrens setzt man je Mol Sulfonylguanidin-Derivat der Formel (II) im allgemeinen zwischen 1 und 5 Mol, vorzugsweise zwischen 1 und 3 Mol einer Verbindung der Formel (III) und gegebenenfalls zwischen 0,01 und 5 Mol, vorzugsweise zwischen 0,1 und 3 Mol einer Base ein.

Die Reaktionskomponenten werden gewöhnlich bei Raumtemperatur zusammengegeben und das Reaktionsgemisch wird bis zum Reaktionsende gerührt. Soweit die Produkte hierbei kristallin anfallen, können sie durch Absaugen isoliert werden. Andernfalls wird eingeengt und mit einem geeigneten Lösungsmittel zur Kristallisation gebracht.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u> Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe können zur selektiven Bekämpfung mono- und dikotyler Unkräuter in mono- und dikotylen Kulturen insbesondere in dikotylen Kulturen wie z.B. Baumwolle, eingesetzt werden. [Die erfindungsgemäßen Wirkstoffe zeigen auch eine fungizide Wirkung gegen Pyricularia oryzae am Reis.]

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. N-(2-Benzthiazolyl)-N,N'-dimethyl-harnstoff, 3-(3-Chlor-4-methylphenyl)-1,1-dimethylharnstoff, 3-(4-isopropylphenyl)-1,1-dimethylharnstoff, 4-Amino-6-(1,1-dimethyl-ethyl)-3-methylthio-1,2,4-triazin-5(4H)-on, 4-Amino-6-(1,1-dimethyl-ethyl)-3-ethylthio-1,2,4-triazin-5(4H)-on, 1-Amino-6-ethylthio-3-(2,2-dimethylpropyl)-1,3,5-triazin-2,4-(1H, 3H)-dion, 4-Amino-3-methyl-6-phenyl-1,2,4-triazin-5(4H)-on, 2-Chlor-4-ethylamino-6-isopropyl-amino-1,3,5-triazin, das R-Enantiomere des 2-/4-(3,5-Dichlor-pyridin-2-oxy)-phenoxy/-propionsäure-(trimethylsilyl)-methylesters, das R-Enantiomere des 2-/4-(3,5-Dichlorpyridyl-2-oxy)-phenoxy/-propionsäure-(2-benzyloxy)-ethylesters, 2,4-Dichlorphenoxyessigsäure, 2-(2,4-Dichlorphenoxy)-propionsäure, 4-Chlor-2-methyl-phenoxy-essigsäure, 2-(2-Methyl-4-chlor-phenoxy)-propionsäure, 3,5-Dijod-4-hydroxy-benzonitril, 3,5-Dibrom-4-hydroxy-benzonitril sowie Diphenylether und Phenylpyridazine, wie z.B. Pyridate. Einige Mischungen zeigen überraschenderweise auch synergistische Wirkung.

Auch eine Mischung mit anderen bekannten Wirkstoffen, wie fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele

Beispiel 1

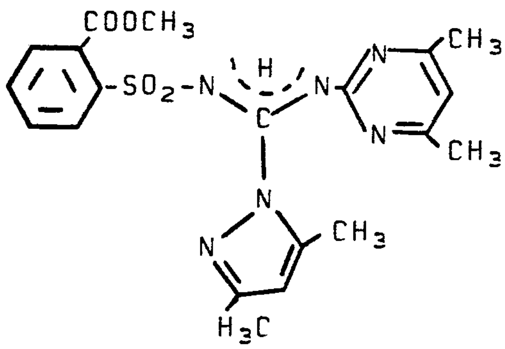

Eine Mischung aus 5,9 g (0,01 Mol) N'-(4,6-Dimethyl-pyrimidin-2-yl)-N''-methoxy-N'',N'''-(2-methoxy-carbonyl-benzolsulfonyl)-guanidin, 1,1 g (0,011 Mol) 3,5-Dimethyl-pyrazol, 3,0 g (0,03 Mol) Triethylamin und 30 ml Aceton wird 2 Tage bei 20°C gerührt. Das kristallin angefallene Produkt wird durch Absaugen isoliert.

Man erhält 2,7 g (61% der Theorie) der Verbindung der oben angegebenen Strukturformel vom Schmelzpunkt 164°C.

Analog können die in der nachstehenden Tabelle 1 aufgeführten Verbindungen der Formel (I) hergestellt werden:

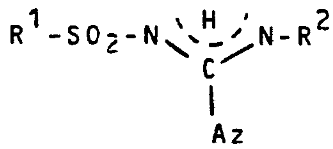

Tabelle 1

| Bsp. Nr. | $R^1$ | $R^2$ | Az | Schmelzpunkt (°C) |
|---|---|---|---|---|
| 2 | 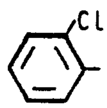 | 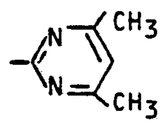 | 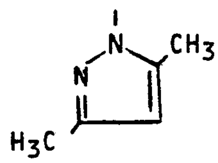 | 197 |
| 3 | 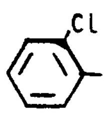 | 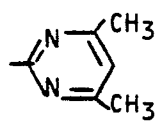 | 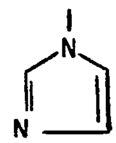 | 214 |
| 4 | 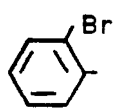 | 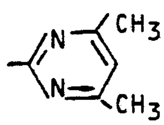 | 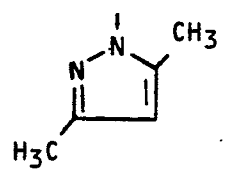 | 198 |
| 5 | 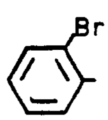 | 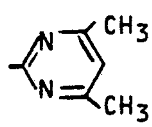 | 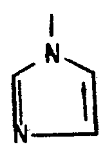 | 178 |

Tabelle 1 (Fortsetzung)

| Bsp. Nr. | $R^1$ | $R^2$ | Az | Schmelzpunkt (°C) |
|---|---|---|---|---|
| 6 | 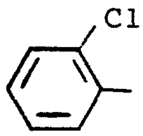 | 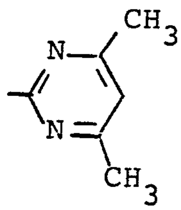 | 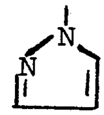 | 162 |
| 7 | 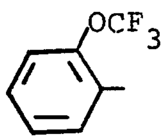 | 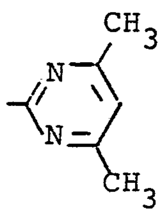 | 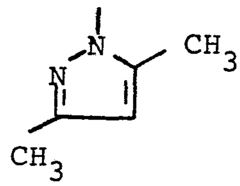 | 196 |
| 8 | 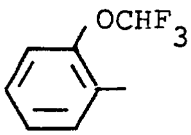 | 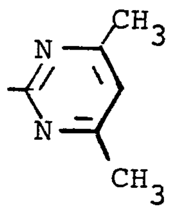 | 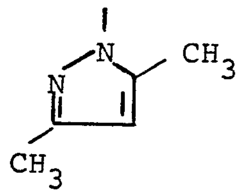 | 183 |
| 9 | 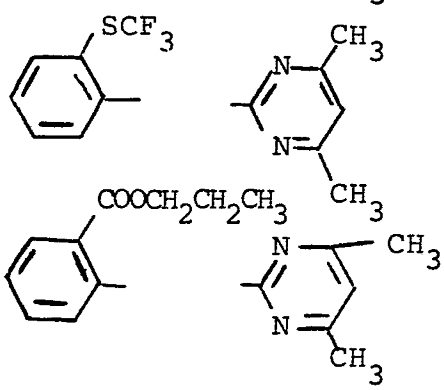 | | 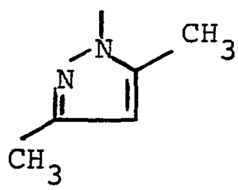 | 220 (Zers.) |
| 10 | | | 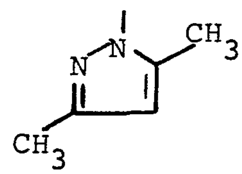 | 103 |
| 11 | 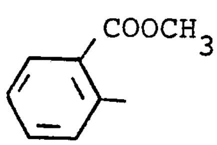 | 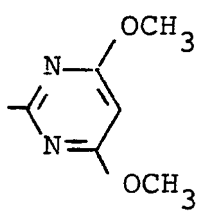 | 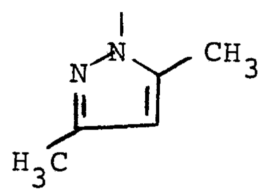 | 166 |

Tabelle 1 (Fortsetzung)

| Bsp. Nr. | $R^1$ | $R^2$ | Az | Schmelzpunkt (°C) |
|---|---|---|---|---|
| 12 | 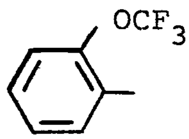 | 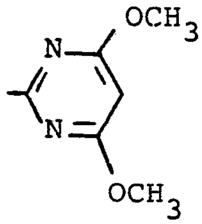 | 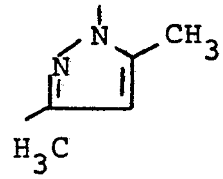 | 101 |
| 13 | 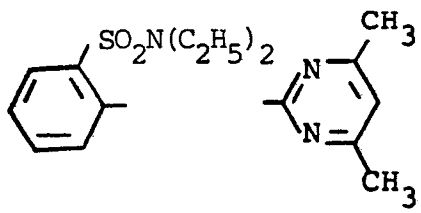 | | 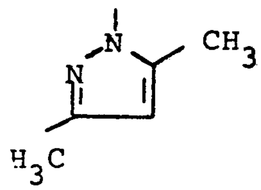 | 149 |
| 14 | 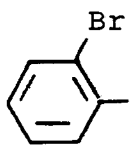 | 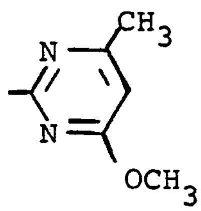 | 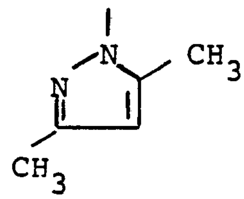 | 142 |

Beispiel 1a

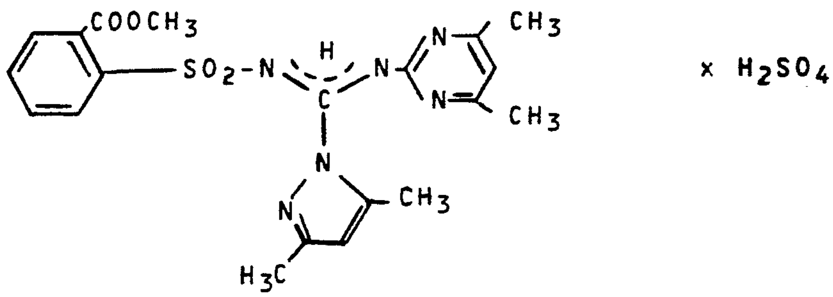

Eine Mischung aus 2,9 g (0,0066 Mol) des Produktes aus Beispiel1, 0,8 g (0,008 Mol) Schwefelsäure und 20 ml Aceton wird 4 Stunden bei 20°C gerührt. Dann wird das kristallin angefallene Produkt durch Absaugen isoliert.

Man erhält 1,8 g (51% der Theorie) des Produktes der oben angegebenen Formel vom Schmelzpunkt 182°C.

Beispiel 1b

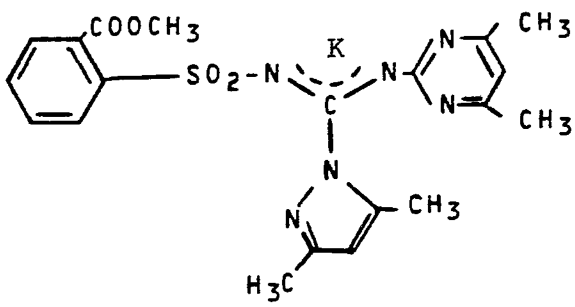

Eine Lösung von 0,5 g (0,006 Mol) Kalium-ethanolat in 15 ml Ethanol wird zu einer Mischung aus 2,6 g (0,006 Mol) des Produktes aus Beispiel 1 und 15 ml Ethanol tropfenweise gegeben. Die Mischung wird ca. 20 Stunden bei 20°C gerührt und das kristallin angefallene Produkt durch Absaugen isoliert.

Man erhält 1,7 g (60 % der Theorie) des Produktes der oben angegebenen Formel vom Schmelzpunkt 143°C.

Analog Beispiel 1b erhält man:

Beispiel 1c

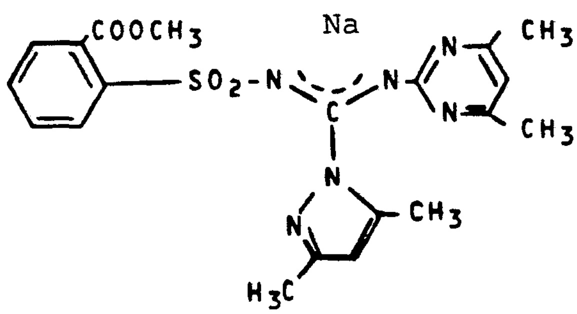

Schmelzpunkt: 138°C

Beispiel 4a

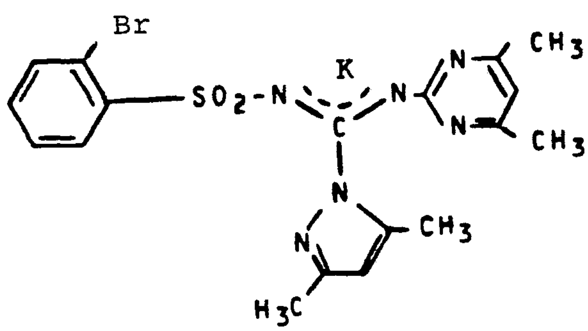

Schmelzpunkt: 181°C

Herstellung von Ausgangsstoffen der Formel (II)

Beispiel (II-1)

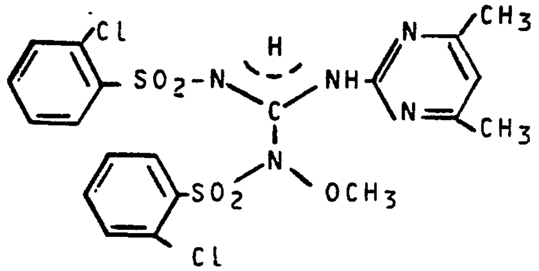

Eine Mischung aus 29,4 g (0,15 Mol) N'-(4,6-Dimethyl-pyrimidin-2-yl)-N''-methoxy-guanidin, 63,6 g (0,3 Mol) 2-Chlor-benzolsulfonsäurechlorid und 150 ml Pyridin wird 2 Tage bei 20°C gerührt. Nach weitgehendem Abdestillieren des Pyridins im Wasserstrahlvakuum wird der Rückstand mit 200 ml Wasser versetzt und mit 200 ml Methylenchlorid extrahiert. Die organische Phase wird abgetrennt, getrocknet und eingeengt. Der Rückstand wird durch Digerieren mit Ethanol zur Kristallisation gebracht.

Man erhält 41,2 g (51% der Theorie) N'-(4,6-Dimethyl-pyrimidin-2-yl)-N''-methoxy-N''-N'''-bis-(2-chlor-benzolsulfonyl)-guanidin vom Schmelzpunkt 164°C bis 166°C.

Analog können die in der nachstehenden Tabelle 2 aufgeführten Verbindungen der Formel (II) hergestellt werden:

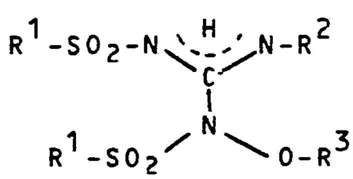

(II)

Tabelle 2

| Bsp. Nr. | $R^1$ | $R^2$ | $R^3$ | Schmelzpunkt (°C) |
|---|---|---|---|---|
| II-2 | 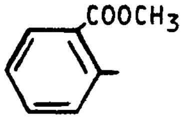 | 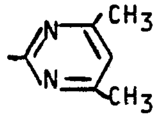 | $CH_3$ | 165 |
| II-3 | 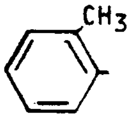 | 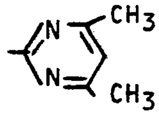 | $CH_3$ | 129 |
| II-4 | 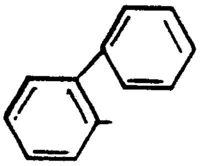 | 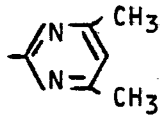 | $CH_3$ | 171 |
| II-5 | 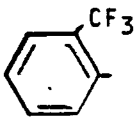 | 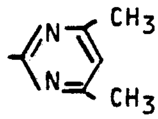 | $CH_3$ | 121 |
| II-6 | 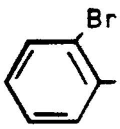 | 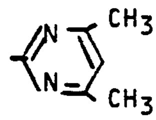 | $CH_3$ | 147 |
| II-7 | 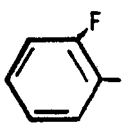 | 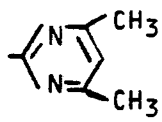 | $CH_3$ | 166 |
| II-8 | 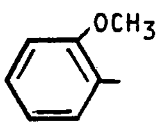 | 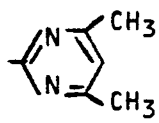 | $CH_3$ | 196 |
| II-9 | 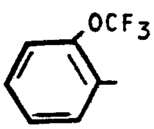 | 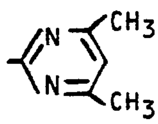 | $CH_3$ | 158 |
| II-10 | 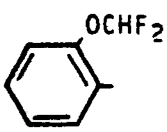 | 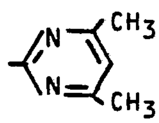 | $CH_3$ | 163 |

Tabelle 2 - Fortsetzung

| Bsp. Nr. | $R^1$ | $R^2$ | $R^3$ | Schmelzpunkt (°C) |
|---|---|---|---|---|
| II-11 | 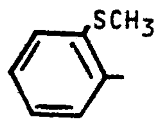 | 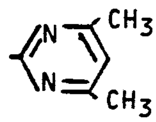 | $CH_3$ | 155 |
| II -12 | 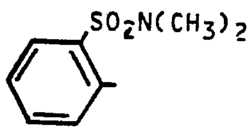 | 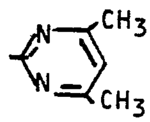 | $CH_3$ | 138 |
| II-13 | 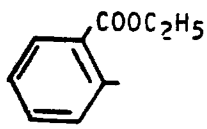 | 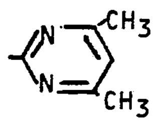 | $CH_3$ | 106 |
| II-14 | 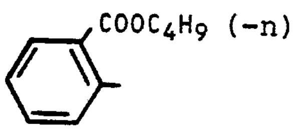 | 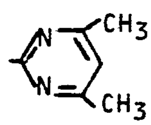 | $CH_3$ | 104 |
| II-15 | 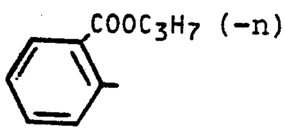 | 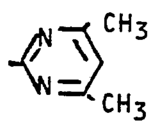 | $CH_3$ | 134 |
| II-16 | 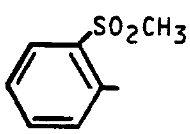 | 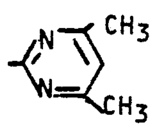 | $CH_3$ | 155 |
| II-17 | 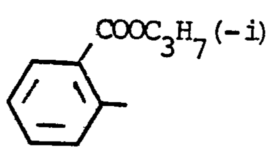 | 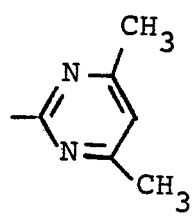 | $CH_3$ | 65 |
| II-18 | 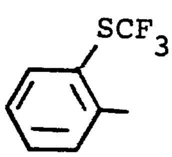 | 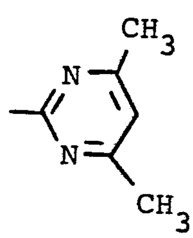 | $CH_3$ | 182-183 |

## Herstellung von Ausgangsstoffen der Formel (IV)

### Beispiel (IV-1)

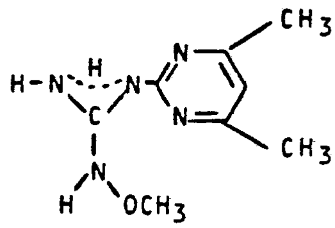

Eine Mischung aus 109 g (0,67 Mol) O-Methylhydroxylamin-Hydrochlorid, 99 g (0,67 Mol) 2-Cyanamino-4,6-dimethyl-pyrimidin und 600 ml Ethanol wird 7 Stunden unter Rückfluß zum Sieden erhitzt. Anschließend wird der Alkohol im Wasserstrahlvakuum abdestilliert, der Rückstand in heißem Wasser gelöst und diese Lösung zu 100 ml konzentriertem Ammoniak gegeben. Das auskristallisierte Produkt wird abgesaugt und aus Ethanol umkristallisiert.

Man erhält 71,8 g (55 % der Theorie) N'-(4,6-Dimethyl-pyrimidin-2-yl)-N''-methoxy-guanidin vom Schmelzpunkt 134°C bis 136°C.

Herstellung von Ausgangsstoffen der Formel (V)

Beispiel (V-1)

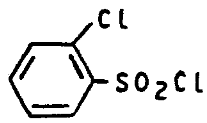

295 ml Phosphorylchlorid ('Phosphoroxychlorid') werden bei 20°C bis 30°C tropfenweise zu einer Mischung aus 172 g (0,8 Mol) 2-Chlor-benzolsulfonsäure-Natriumsalz, 300 ml Acetonitril und 300 ml Sulfolan gegeben. Das Reaktionsgemisch wird 4 Stunden bei 70°C gerührt, anschließend auf 5°C abgekühlt und mit Eiswasser verdünnt. Nach Extrahieren mit Petrolether, Waschen der Extraktionslösung mit Wasser, Trocknen, Filtrieren und Einengen wird das im Rückstand verbliebene Produkt durch Vakuumdestillation gereinigt.

Man erhält 117 g (70 % der Theorie) 2-Chlor-benzolsulfonsäurechlorid vom Siedepunkt 110°C/1,1 mbar.

Beispiel (V-2)

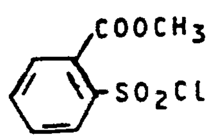

75,5 g (0,5 Mol) 2-Aminobenzoesäuremethylester werden in 176 ml konzentrierte Salzsäure und 100 ml Essigsäure gelöst. Hierzu wird bei 0°C eine Lösung von 34,4 g Natriumnitrit in 70 ml Wasser getropft. Nach 15-minütigem Nachrühren wird das Reaktionsgemisch langsam zu einer auf 0°C gekühlten gesättigten Lösung von Schwefeldioxid in 450 ml Essigsäure gegeben. Nach Entfernung des Kühlbades wird bis zum Ende der Gasentwicklung gerührt, wobei 10 g Kupfer(II)-chlorid portionsweise eingetragen werden. Nach Verdünnen mit Eiswasser, Extrahieren mit Methylenchlorid, Waschen der Extraktionslösung mit Wasser, Trocknen, Filtrieren und Einengen wird das im Rückstand verbliebene Produkt durch Vakuumdestillation gereinigt.

Man erhält 45 g (38% der Theorie) 2-Methoxycarbonyl-benzolsulfonsäurechlorid vom Siedepunkt 150°C/1,33 mbar.

Analog können die in der nachstehenden Tabelle 3 aufgeführten Verbindungen der Formel (V) hergestellt werden:

$$R^1-SO_2-Cl \qquad (V)$$

Tabelle 3

| Bsp.-Nr. | $R^1$ | Siedepunkt/Druck |
|---|---|---|
| V -3 | 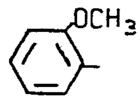 | (Oel,Zersetzung bei Destillation) |
| V -4 | 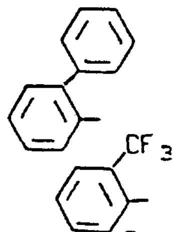 | [Schmelzpunkt:100°C] |
| V -5 | | (Oel) |
| V -6 | 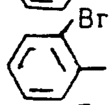 | 142°C/4 mbar |
| V -7 | 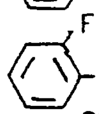 | 106°C/4 mbar |
| V -8 | 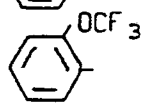 | [Schmelzpunkt:32°C] |
| V -9 | 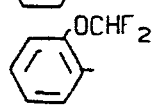 | (Oel, Zersetzung bei Destillation) |
| V -10 | 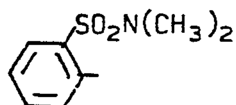 | [Schmelzpunkt:103°C] |
| V -11 | 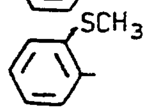 | (Oel, Zersetzung bei Destillation) |
| V -12 | 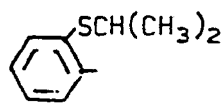 | 90°C/1,33 mbar |
| V -13 | 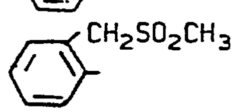 | [Schmelzpunkt:120°C] |
| V -14 | 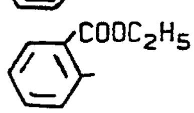 | 155°C/5,32 mbar |

Tabelle 3 (Fortsetzung)

| Bsp. Nr. | $R^1$ | Siedepunkt/Druck |
|---|---|---|
| V-15 | 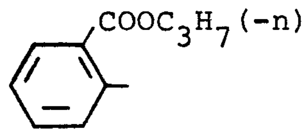 | |
| V-16 | 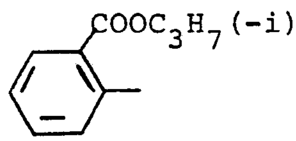 | |
| V-17 | 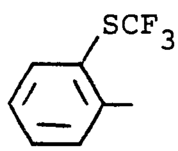 | 41-43°C |
| V-18 | 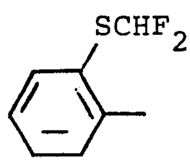 | |

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Bei diesem Test zeigen z.B. die folgenden Verbindungen der Herstellungsbeispiele eine ausgezeichnete Wirksamkeit: (1),(2), (4) und (5)

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Bei diesem Test zeigen z.B. die folgenden Verbindungen der Herstellungsbeispiele eine ausgezeichnete Wirksamkeit : (1), (2), (4) und (5).

Patentansprüche

1) Amidinoazole der allgemeinen Formel (I)

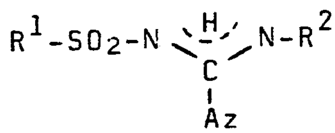

(I)

in welcher

$R^1$ für einen gegebenenfalls substituierten Rest aus der Reihe Alkyl, Aralkyl, Aryl und Heteroaryl steht,

$R^2$ für einen gegebenenfalls substituierten und/oder gegebenenfalls anellierten sechsgliedrigen aromatischen Heterocyclus, welcher wenigstens ein Stickstoffatom enthält, steht und

Az für einen gegebenenfalls substituierten Azolrest, d. h. einen fünfgliedrigen aromatischen Heterocyclus, welcher wenigstens ein Stickstoffatom enthält, steht,

sowie Metall-Salze von Verbindungen der Formel (I) und Addukte von Verbindungen der Formel (I) mit starken Säuren.

2) Verfahren zur Herstellung von Amidinoazolen der allgemeinen Formel (I) gemäß Anspruch 1, dadurch gekennzeichnet, daß man Sulfonylguanidin-Derivate der Formel (II)

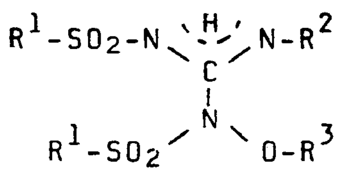

(II)

in welcher

$R^1$ und $R^2$ die oben angegebenen Bedeutungen haben und

$R^3$ für einen gegebenenfalls substituierten Kohlenwasserstoff-Rest steht,

mit Verbindungen der Formel (III)

M-Az (III)

in welcher

Az die oben angegebene Bedeutung hat und

M für Wasserstoff oder ein Metalläquivalent steht,

gegebenenfalls in Gegenwart von Basen und in Gegenwart von Verdünnungsmitteln bei Temperaturen zwischen 0 °C und 100 °C umsetzt und gegebenenfalls die hierbei erhaltenen Produkte der Formel (I) mit Metallverbindungen bzw. mit Säuren behandelt.

3. Herbizide Mittel, gekennzeichnet durch einen Gehalt an mindestens einem Amidinoazol der allgemeinen Formel (I) gemäß Anspruch 1.

4) Verwendung von Amidinoazolen der allgemeinen Formel (I) gemäß Anspruch 1 zur Bekämpfung von unerwünschtem Pflanzenwachstum.

5) Verfahren zur Herstellung von herbiziden Mitteln, dadurch gekennzeichnet, daß man Amidinoazole der allgemeinen Formel (I) gemäß Anspruch 1 mit Streckmitteln und/oder oberflächenaktiven Mitteln vermischt.

Europäisches Patentamt

**EUROPÄISCHER RECHERCHENBERICHT**

0 173 323

EP 85110837.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X,P | EP - A1 - 0 121 082 (BAYER)<br>* Formel I; Seite 5; Ansprüche 1,3,4; Beispiel 129 *<br>-- | 1,2,3,4,5 | C 07 D 401/12<br>C 07 D 401/14<br>C 07 D 403/12<br>C 07 D 403/14<br>C 07 D 405/14<br>C 07 D 409/14<br>C 07 D 413/12<br>C 07 D 413/14<br>C 07 D 417/12<br>C 07 D 417/14<br>C 07 D 419/14<br>C 07 D 471/00<br>C 07 D 487/00<br>C 07 D 491/00<br>C 07 D 493/00<br>C 07 D 495/00<br>C 07 D 498/00<br>C 07 D 513/00<br>A 01 N 47/44 |
| A | EP - A1 - 0 117 014 (E.I. DU PONT)<br>* Zusammenfassung; insbesondere Formel II *<br>---- | 1,3,4 | |
| | | | **RECHERCHIERTE SACHGEBIETE (Int. Cl.4)** |
| | | | C 07 D 401/00<br>C 07 D 403/00<br>C 07 D 405/00<br>C 07 D 409/00<br>C 07 D 413/00<br>C 07 D 417/00<br>C 07 D 419/00 |

Der vorliegende Recherchenbericht wurde für alle Patentansprüche erstellt.

| Recherchenort | Abschlußdatum der Recherche | Prüfer |
|---|---|---|
| WIEN | 04-11-1985 | HAMMER |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82